# EUROPEAN PATENT APPLICATION

(11) **EP 4 455 292 A1**
(43) Date of publication of application: **30.10.2024**
(21) Application number: 23170142.6
(22) Date of filing: 26.04.2023
(51) Int. Cl.: C12N 15/63, C12N 15/65, C12N 15/70, C12N 15/78

(54) **A METHOD FOR DETECTING ECTOINE**

(71) Applicant: Kaunas University of Technology, 44029 Kaunas (LT)
(72) Inventor: Malys, Naglis, Kaunas (LT); Matulis, Paulius, Kaunas (LT)
(74) Representative: Petniunaite, Jurga

(57) **Abstract**

Ectoine-responsive biosensor comprising a recombinant host cell and DNA polynucleotide sequences which encode a transcription factor EutR and inducible promoter operably linked to a coding region, wherein the coding region encodes a reporter gene. The transcription factor through the binding to the inducible promoter regulates transcription of the coding region. The biosensor comprises DNA polynucleotide sequences in a plasmid construct. The use of biosensor comprises a method for detecting ectoine and measuring its concentration.

## Description

### FIELD OF THE INVENTION

The present invention relates to a biosensor and a method for the detection of ectoine and measuring concentration of this compound.

### BACKGROUND OF THE INVENTION

Heterocyclic amino acids such as ectoine or hydroxyectoine has excellent function-preserving properties that attracted their use in a wide range of products for skin care and cosmetics [Czech et al (2018) Genes 9(4), 177]. Commercial demand for ectoines has led to a development of industrial production process that utilizes Halomonas elongata as a microbial cell factory via ectoine milking and further improved by identification of ectoine leaky strains which do not require ectoine milking procedures [Kunte et al (2014) Curr. Biotechnol. 3, 10-25]. In aforementioned research their method of direct bacterial system application for ectoine detection involved its property of osmoprotectant. E. coli in combination of growth under high salt conditions and β-galactosidase enzyme activity displayed visible blue hallo - signifying growth only in presence of ectoine. This approach while usable for leaky ectoine producer screening is limited in non-leaky strain identification and is relatively slow as experiment requires around 24 hours of incubation under mixed culture conditions.

The closest prior art document [Chen et al (2015) Metab. Eng. 30, 149-155] discloses AraC-P_{BAD} based genetically encoded reporter system developed by random mutagenesis of AraC transcription regulator this research final result is similar to ours however, the transcription factor generated by random mutagenesis with typical P_{BAD} binding site can't be used in *E. coli* strains harboring natural arabinose inducible systems. Additionally, when comparing systems in E. *coli* expression organism, our sensor has advantages such as, having around 1000 fold lower minimal ectoine concentration needed for observable induction, with our being as low as 1 µM of ectoine and theirs being around 10 mM of ectoine, meaning our sensor has more appropriate sensitivity to be directly usable in biological systems. Finally, our sensor does not display noticeable reporter gene expression in uninduced state, while according to data, their system has more than 150000 GFP fluorescence units at uninduced state, which would qualify their system highly leaky in context of protein expression regulation.

To further develop methods for sensitive and rapid quantification of heterocyclic amino acids such as ectoine or hydroxyectoine from microbial production strains, new approaches and methodologies are being developed. Particularly, the screening for improved production strains requires testing and analysis of strain libraries ranging up to billion different variants with the larger library enabling a higher probability of identifying a variant with improved production. The analysis by using high performance liquid chromatography (HPLC), which is commonly used for ectoine detection and quantification, suffers from low throughput with only a few hundred samples analysed in a week. Besides, intracellular detection of ectoines requires extraction with organic solvents, several steps of liquid handling and concentration before the sample is ready for HPLC analysis. The application of HPLC based methodology draws significant cost that limits the scale of the variant screening. Consequently, there is a need for a tool and method that can be applied for the high-throughput and low-cost heterocyclic amino acids' screening.

Transcription factor-based biosensors responding to chemical compounds are valuable tools contributing to the diagnostics and assisting the microbial cell factory development. Importantly, they can be effectively used for high-throughput screening to facilitate improved chemical compounds' biosynthesis. Moreover, they can be used to control gene/pathway expression, build regulatory networks, screen or select enzymes, pathways or even strains, and importantly to detect and quantify endogenous and exogenous concentrations of chemical compounds/metabolites. Importantly, transcription factor, a key player of such biosensors, can be directly controlled by the effector (chemical compound/metabolite) activating or repressing the RNA polymerase on a given promoter. Although, transcription repressor/activator proteins responding to chemical compounds/metabolites are numerous in nature, the number of compounds that can be detected and quantified using biosensors is still limited, whereas heterocyclic amino acids' biosensor has not been developed yet. The present invention addresses the need for biosensor and method for detecting ectoine.

### SUMMARY OF THE INVENTION

According to a first aspect of the invention a biosensor comprising host cell and transcription factor-based inducible gene expression that can be used for the accurate detection of exogenous heterocyclic amino acids in liquid or solid media and in vivo detection of endogenously produced heterocyclic amino acids within a host is disclosed. Microorganisms are highly adept at sensing and responding to small-molecules in their environment. For example, transcription factors that respond to the presence of heterocyclic amino acids can modulate expression of reporter gene downstream of the transcription factor's cognate promoter comprising an inducible promoter composed of an operator and a promoter. By monitoring the expression of the reporter gene, the heterocyclic amino acid concentration can be readily measured.

The biosensor uses a transcription factor-based inducible gene expression system to drive the expression of a reporter gene, wherein when the inducible system is not activated; the expression of the reporter is absent or very low. The inducible system is activated in dose-dependent manner in the presence of ectoine.

Thus, in some embodiments, the invention provides a recombinant host cell comprising a biosensor comprising DNA polynucleotide sequences which encode a transcription factor and inducible promoter operably linked to a coding region, wherein the coding region encodes a reporter gene. The transcription factor through the binding to the inducible promoter regulates transcription of the coding region; the extracellular presence of heterocyclic amino acid such as ectoine or hydroxyectoine (effector) modulates the binding of transcription factor to the operator of inducible promoter in dose-dependent manner. More specifically, the transcription factor's binding to the operator of inducible promoter is highly activated by the interaction of host cell-specific, ectoine- or hydroxyectoine-derived compounds, alpha-acetyldiaminobutyric acid (alpha-ADABA) or its catabolic products, with the transcription factor.

In some embodiments, the recombinant host cell comprises DNA polynucleotide sequences encoding the endogenous transcription factor and endogenous inducible promoter operably linked to the heterologous reporter gene. In some embodiments, the recombinant host cell comprises DNA polynucleotide sequences encoding the heterologous transcription factor and heterologous inducible promoter operably linked to the heterologous reporter gene. The heterocyclic amino acid to which the biosensor responds may be an ectoine or hydroxyectoine.

In some embodiments, the host cell further comprises nucleic acid sequences encoding heterocyclic amino acid transporters to transport, e.g., uptake, exogenous heterocyclic amino acid. In some embodiments, the host cell can further comprise nucleic acid sequences encoding enzymes that convert the hydroxyectoine into ectoine, and then the ectoine into alpha-ADABA or its catabolic products.

In some embodiments of the invention, the transcription factor itself comprises the protein moiety that binds the effector or its catabolic product, e.g., the transcription factor may be a Pseudomonas putida EutR transcription factor. In some embodiments, the inducible promoter that is operably linked to a reporter gene to which the EutR binds comprises a naturally-occurring Pseudomonas putida OeutB operator and PeutB promoter or a synthetic *OeutB operator and Pseudomonas putida PeutB promoter.

A recombinant host cell comprising a heterocyclic amino acid-biosensor system of the invention may be any kind of prokaryotic cell. Preferably, the recombinant host cell is Pseudomonas putida or Escherichia coli.

According to another aspect of the invention a method for detecting a heterocyclic amino acid ectoine is disclosed. The method comprises providing a recombinant host cell that comprises a heterocyclic amino acid-biosensor described herein and detecting expression of the reporter gene.

In some embodiments, the method detects the presence of heterocyclic amino acid that is exogenous and endogenous. In some embodiments, the recombinant host cell is in contact with a mixture, e.g., cell culture media or supernatant, which are being analysed for the presence of ectoine.

According to yet another aspect of the invention a recombinant host cell capable of transcribing RNA is disclosed. The recombinant host cell comprises: a transcription factor that can bind to and modulate an activity of promoter; a transcription factor that binds an ectoine, hydroxyectoine or their catabolic product; the inducible promoter that comprises an operator and a promoteris; and the inducible promoter, which activity is modulated by the transcription factor and which is operably linked to a nucleic acid sequence that encodes a heterologous reporter gene.

### DESCRIPTION OF DRAWINGS

The drawings set forth herein are illustrative of embodiments of the invention and are not meant to limit the scope of the invention as encompassed by the claims.

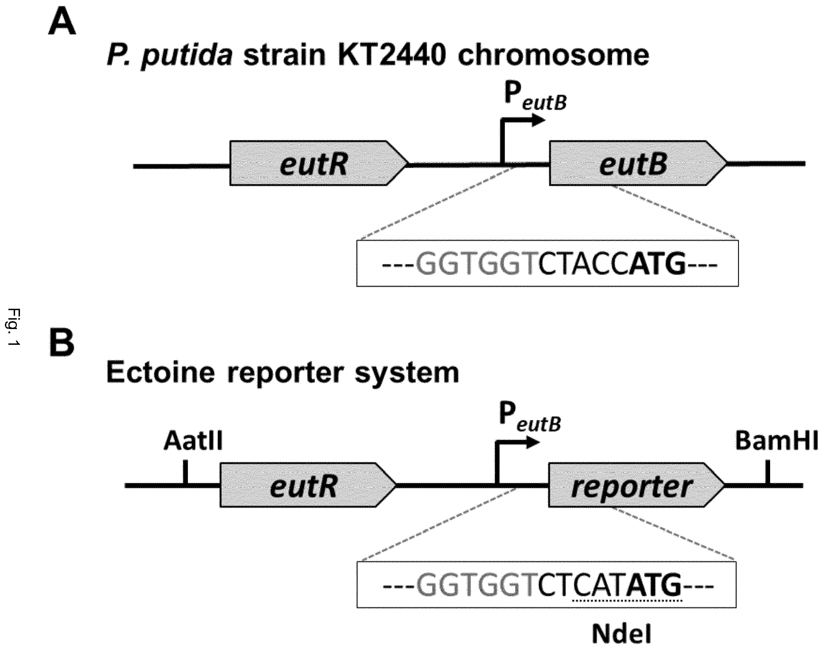

Fig. 1 shows a schematic illustration of the ectoine-inducible system EutR/PeutB from P. putida strain KT2440. The system includes the transcription factor gene eutR and the eutB gene, which encodes a hydroxyectoine utilization dehydratase involved in ectoine metabolism. As a reporter system, the transcription factor gene eutR, the intergenic region eutR-eutB with inducible promoter PeutB, and the reporter gene are flanked by the restriction sites Aatll and BamHI for cloning into the modular pBRC1 [Augustiniene and Malys (2022) Sci. Rep.12(1), 2123]. The inducible system including the transcription factor gene eutR and the intergenic region eutR-eutB with inducible promoter PeutB,are flanked by the restriction sites Aatll and Ndel for easy exchange of modified inducible system variants.

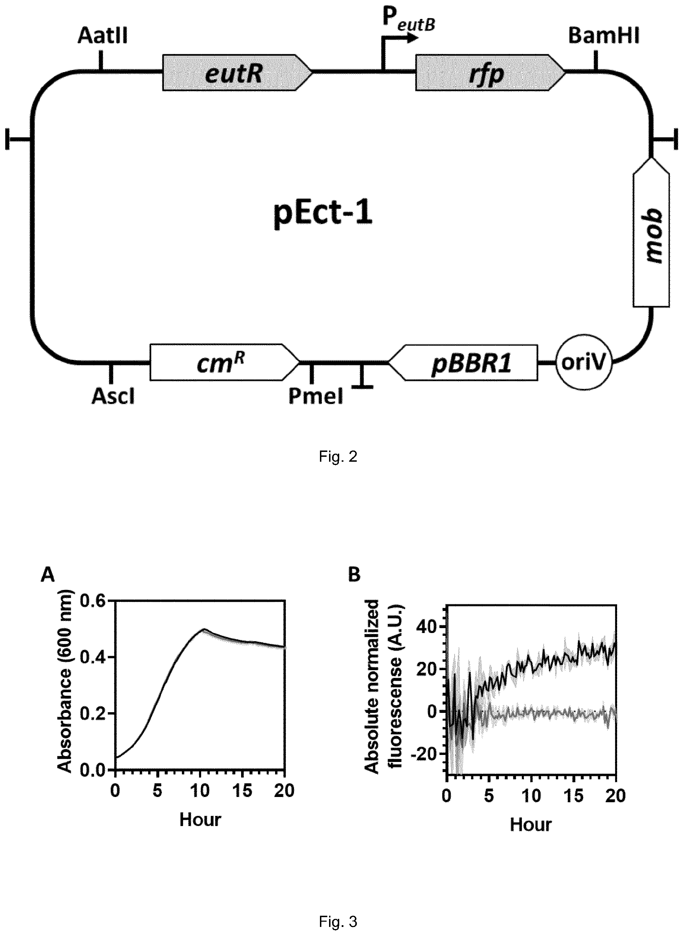

Fig. 2 shows schematic illustration of the pEct-1. The reporter gene rfp is placed under the control of the ectoine-inducible system EutR/PeutB system. Chloramphenicol resistance gene cmR, mobilization gene mob, and the origin of replication oriV and associated pBBR1 are indicated.
Fig. 3 shows E. coli-based biosensor's cell growth and reporter response kinetics. (A) The cell absorbance (at 600nm) of E. coli harbouring the plasmid pEct-1 in the presence of 0 or 1 mM ectoine that was supplemented at 0 hour time point. Cells were cultivated in MM medium. (B) Absolute normalized fluorescence profiles over time of cells grown in MM medium in the presence of 0 or 1 mM ectoine supplemented at 0 hour time point. In all cases, the average of three biological replicates is shown with error bars represented in lighter colour.

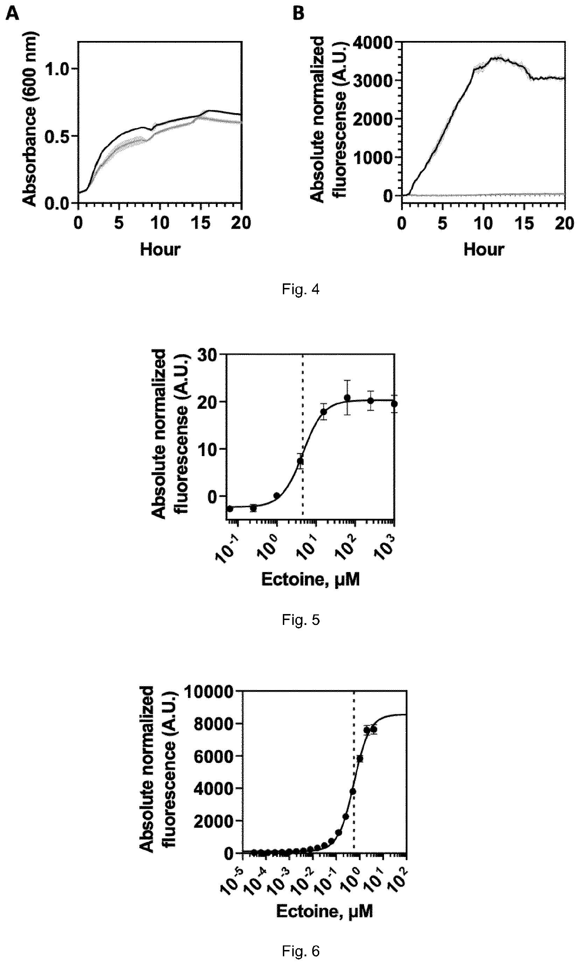

Fig. 4 shows P. putida-based biosensor's cell growth and reporter response kinetics. (A) The cell absorbance (at 600nm) of E. coli harbouring the plasmid pEct-1 in the presence of 0 or 1 mM ectoine that was supplemented at 0 hour time point. Cells were cultivated in MM medium. (B) Absolute normalized fluorescence profiles over time of cells grown in MM medium in the presence of 0 or 1 mM ectoine supplemented at 0 hour time point. In all cases, the average of three biological replicates is shown with error bars represented in lighter colour.
Fig. 5 shows a dose-response dynamics of the E. coli-based ectoine biosensor. Cells harbouring the plasmid pEct-1 were cultivated in MM medium supplemented with various concentrations of ectoine (0, 0.24, 0.98, 3.9, 15.6, 62.5, 250 and 1000 µM) at early exponential growth phase. Absorbance and fluorescence were measured 10 hours after supplementation of ectoine.
Fig. 6 shows a dose-response dynamics of the P. putida-based ectoine biosensor. Cells harbouring the plasmid pEct-1 were cultivated in MM medium supplemented with various concentrations of ectoine (0, 0.24, 0.98, 3.9, 15.6, 62.5, 250 and 1000 µM) at early exponential growth phase. Absorbance and fluorescence were measured at 10-hour point after supplementation with ectoine.

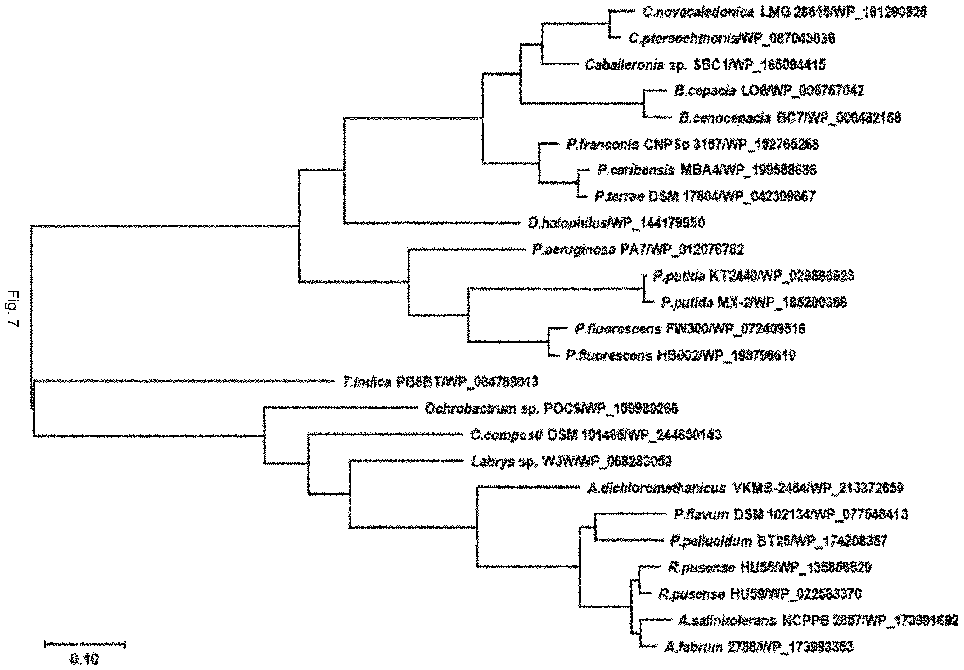

Fig. 7 shows a phylogenetic tree of MocR/GabR sub-family of GntR-type transcription factor. Neighbour-Joining tree with 40 homologous MocR/GabR sub-family transcription factors from species belonging to the genus Pseudomonas was aligned based on the respective protein sequences. Branch lengths correspond to the relative divergence in amino acid sequences. EutR from P. putida KT2440 has an accession number WP_029886623. The transcription factors most related to EutR were used for the identification of consensus motif for operator.

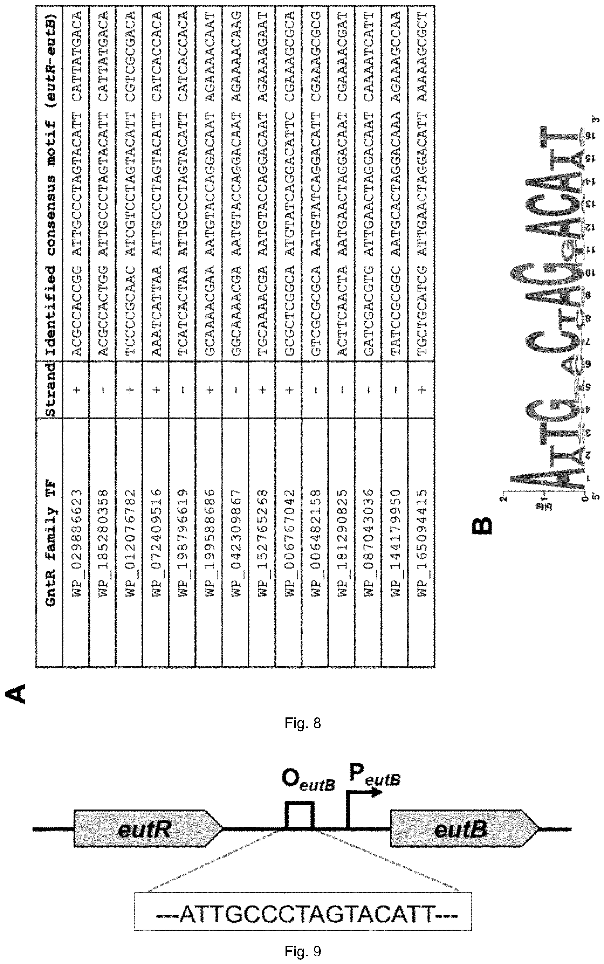

Fig. 8 shows a conservative operator OeutB sequence identified within the intergenic region eutR-eutB downstream of the gene encoding the MocR/GabR sub-family transcription factor. (A) Operator OeutB sequence identified in 12 strains that contain genes encoding EutR homologues with amino acid sequence identity greater than 90%. The operator OeutB sequence corresponding to P. putida KT2440 transcription factor EutR, with accession number WP_029886623, is provided at the top of the list. (B) Operator OeutB sequence logo based on 12 sequences identified in strains that contain genes encoding EutR homologues with amino acid sequence identity greater than 90%.
Fig. 9 shows a schematic representation of the EutR/OPeutB system and the putative operator sequence OeutB.

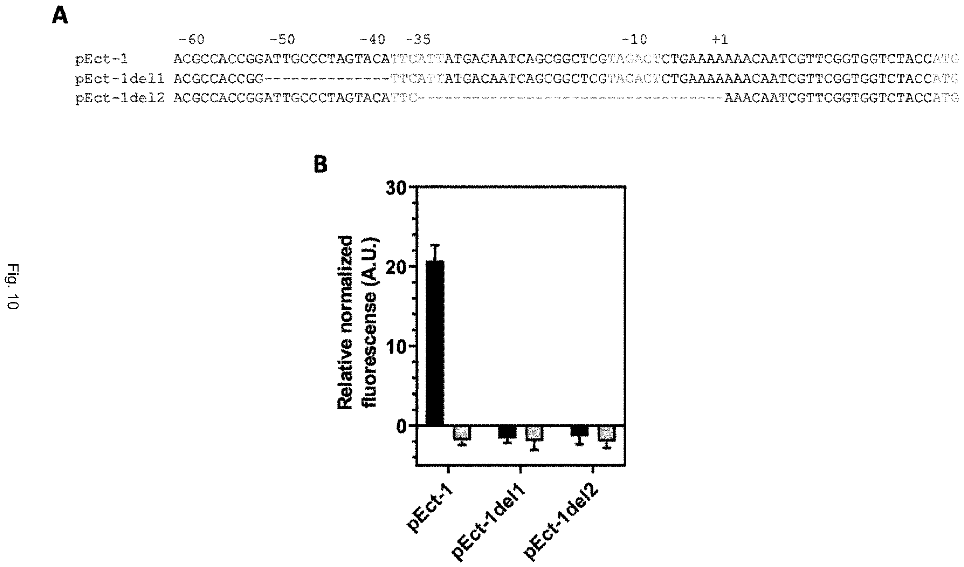

Fig. 10 shows an analysis of regulatory elements in the intergenic region eutR-eutB. (A) A schematic representation of the deletion of nucleotide sequences containing putative OeutB (dell) and PeutB (del2). (B) Analysis of the regulatory elements of the EutR/OPeutB system by measuring fluorescence output using E. coli strains harbouring plasmids pEct-1del1 and pEct-1del2 with corresponding deletions. The plasmid pEct-1 containing unaltered sequence of intergenic region eutR-eutB was used as a control. Cells containing the designated plasmids were cultivated in MM medium in the absence and presence of 1 mM ectoine. The absolute normalized fluorescence was determined 10 hours after supplementation of ectoine. Error bars represent standard deviations of three biological replicates.

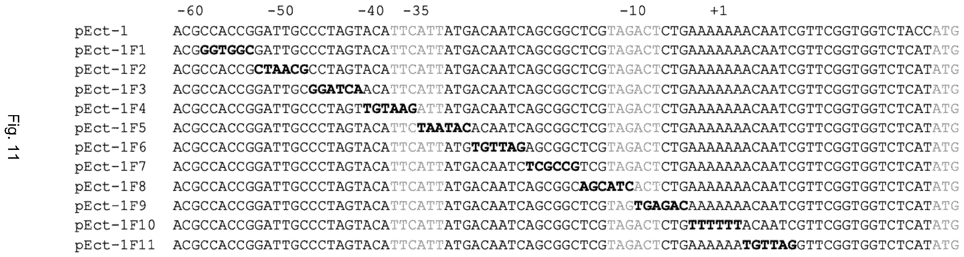

Fig. 11 shows DNA sequence of the EutR/OPeutB system variants F1 to F11 with mutated intergenic region eutR-eutB, where sequence blocks of 6-nucleotide were substituted by complementary sequences.

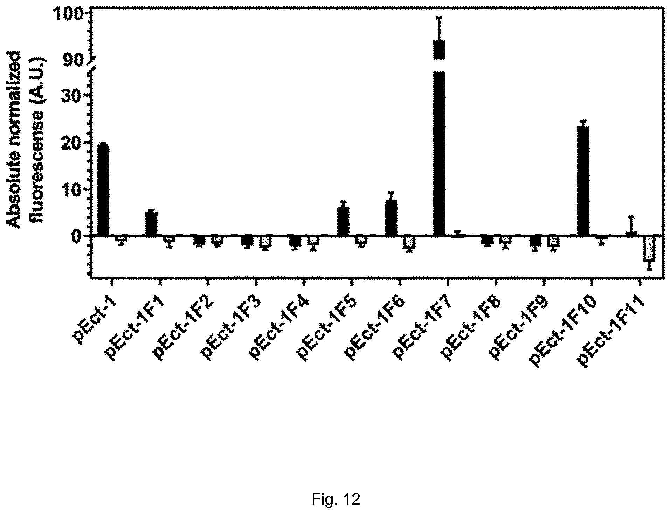

Fig. 12 shows an analysis of the intergenic region eutR-eutB of the EutR/OPeutB system by measuring fluorescence output using E. coli strains harbouring plasmids pEct-1 F1 to pEct-1 F11 with corresponding substitution F1 to F11. The plasmid pEct-1 containing unaltered sequence of intergenic region eutR-eutB was used as a control. Cells containing the designated plasmids were cultivated in MM medium in the presence and absence of 1 mM ectoine. The absolute normalized fluorescence was determined 10 hours after supplementation of ectoine. Error bars represent standard deviations of three biological replicates.

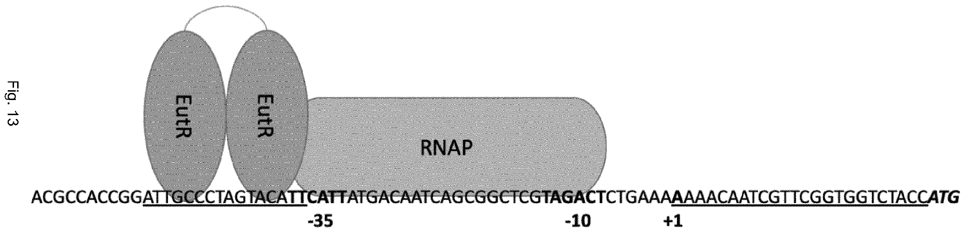

Fig. 13 shows a putative EutR binding site and a regulation of the EutR/OPeutB system. EutR dimer operator's OeutB sequence and regulates RNAP binding to the promoter's PeutB. -10 and -35 sequences (highlighted in bold). TSS and gene's eutB start codon are also highlighted.

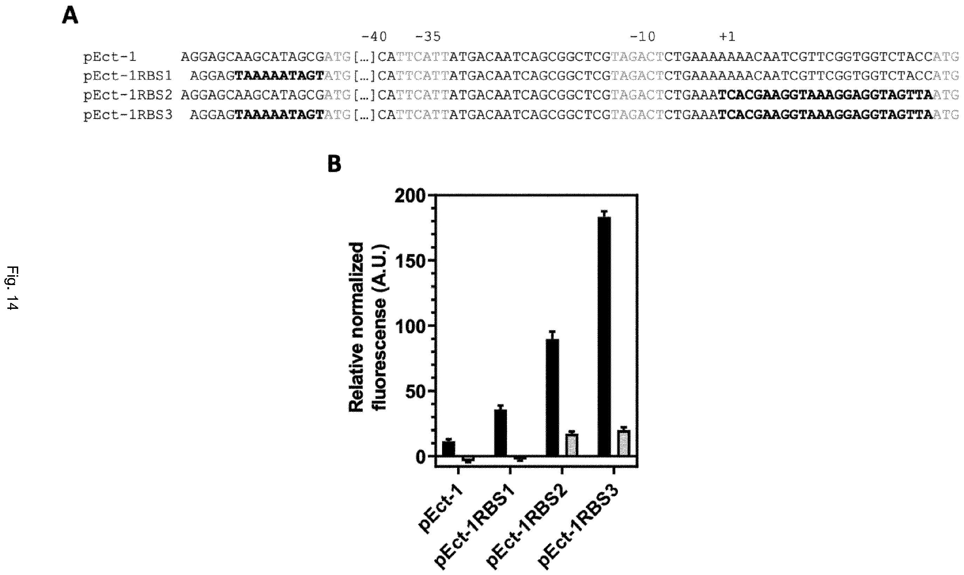

Fig. 14 shows an analysis of altered ribosome binding sites in the intergenic region eutR-eutB. (A) A schematic representation of the alteration of nucleotide sequences including ribosome binding sites upstream of eutR and eutB. (B) Analysis of the alteration of ribosome biding sites of the EutR/OPeutB system by measuring fluorescence output using E. coli strains harbouring plasmids pEct-1-RBS1, pEct-1-RBS2 and pEct-1-RBS1. The plasmid pEct-1 containing unaltered sequence of intergenic region eutR-eutB was used as a control. Cells containing the designated plasmids were cultivated in MM medium in the absence and presence of 1 mM ectoine. The absolute normalized fluorescence was determined 10 hours after supplementation of ectoine. Error bars represent standard deviations of three biological replicates.

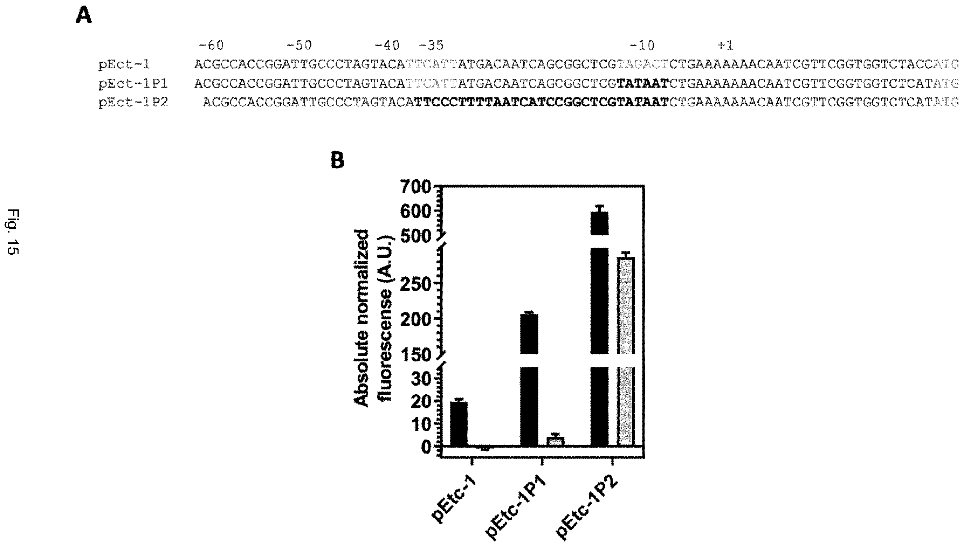

Fig. 15 shows an analysis of altered promoter PeutB in the intergenic region eutR-eutB. (A) A schematic representation of the alteration of nucleotide sequences containing putative promoter PeutB. (B) Analysis of the alteration of the promoter PeutB of the EutR/OPeutB system by measuring fluorescence output using E. coli strains harbouring plasmids pEct-1_P1 and pEct-1-P2. The plasmid pEct-1 containing unaltered sequence of intergenic region eutR-eutB was used as a control. Cells containing the designated plasmids were cultivated in MM medium in the presence and absence of 1 mM ectoine. The absolute normalized fluorescence was determined 10 hours after supplementation of ectoine. Error bars represent standard deviations of three biological replicates.

### DETAILED DESCRIPTION OF THE INVENTION

Before the present invention is described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a carboxylic acid" includes a plurality of such carboxylic acids, and so forth.

In this specification and in the claims that follow, reference will be made to a number of terms that shall be defined to have the following meanings:
The terms "plasmid" or "plasmid construct" refer to a compound and/or composition that can be introduced into a host cell by any suitable method, including but not limited to transduction, transformation, transfection, infection, electroporation, conjugation, and the like; thereby causing the cell to express nucleic acids and/or proteins other than those native to the cell, or in a manner not native to the cell. An "plasmid" or "plasmid construct" contains a sequence of nucleic acids (ordinarily RNA or DNA) to be expressed by the host cell. The plasmids contemplated for use in the present invention include those into which a nucleic acid sequence can be inserted, along with any preferred or required operational elements. Further, the plasmid must be one that can be transferred into a host cell and replicated therein. Plasmids contain restriction sites that have been well documented and that contain the operational elements preferred or required for transcription of the nucleic acid sequence. Such plasmids are well known to those of ordinary skill in the art.

The term "isolated" refers to material that is substantially or essentially free of components that normally accompany it in its native state.

As used herein, the terms "nucleic acid", "nucleotide" and variations thereof shall be generic to polydeoxyribonucleotides (containing 2-deoxy-D-ribose), to polyribonucleotides (containing D-ribose), to any other type of polynucleotide that is an N-glycoside of a purine or pyrimidine base, provided that the polymers contain nucleobases in a configuration that allows for base pairing and base stacking, as found in DNA and RNA.

The term "operably linked" refers to a functional linkage between a nucleic acid expression control sequence (such as a promoter) and a second nucleic acid sequence, wherein the expression control sequence directs transcription of the nucleic acid corresponding to the second sequence.

These and other objects, advantages, and features of the invention will become apparent to those persons skilled in the art upon reading the details of the invention as more fully described below.

In some circumstances the gene expression needs to be activated when the cell is exposed to chemical compound such as ectoine. Furthermore, the inducible gene expression system-based biosensor can be used to monitor and measure the concentration of chemical compound ectoine. The aim of the present invention is to provide an inducible gene expression system which responds to ectoine and offers method for measuring ectoine concentration.

According to a first aspect of the invention an ectoine biosensor is disclosed.

A method for detecting ectoine, the method comprises
providing a recombinant host cell as biosensor,
characterized in that
a first nucleotide sequence of the recombinant host cell is encoding a transcription factor, wherein the transcription factor regulates transcription of the target gene in response to an effector;
a second nucleotide sequence of the recombinant host cell is containing regulatory elements including a DNA-binding site, the operator, transcription of which transcription factor regulates and a promoter;
and a third nucleotide sequence of the recombinant host cell comprises a reporter gene operably linked to the promoter that is activated in response to the effector;
the host cell is cultured under conditions when the effector is exogenously or endogenously present;
the effector interacts with transcription factor by activating transcription of the reporter gene;
the reporter gene is monitored; and
the concentration of ectoine is monitored, where the monitoring of ectoine comprises:
   i) providing a host cell which contains a genetic construct, the construct comprising polynucleotide to be transcribed, wherein the polynucleotide comprises a coding region encoding a target gene operably linked to a transcription factor and regulatory elements;
   ii) exposing the transformed cell to an effector of the transcription factor thereby effecting expression of the target gene wherein the target gene is reporter gene.

The ectoine biosensor comprises a first nucleotide sequence encoding a transcription factor, wherein the transcription factor regulates expression of target gene in response to an effector, a second nucleotide sequence containing regulatory elements and a third nucleotide sequence encoding a target gene that can encode the fluorescent reporter protein, wherein the target gene is expressed in response to the effector and translation (protein synthesis) of the transcription factor.

A nucleotide sequence according to the invention may be DNA (such as cDNA) or RNA (such as mRNA). Preferably, the first, second and third nucleotide sequences referred to herein are the same type of nucleotide sequence, for example, all DNA or all RNA.

Preferably, the first nucleotide sequence and the third nucleotide sequence are divergently orientated, whereas the second nucleotide sequence is located between the first and third sequences. The skilled person would appreciate that divergently orientated genes, are regulated by separate cis regulatory elements located in the second nucleotide sequence. The first nucleotide sequence and/or the third nucleotide sequence may be operatively linked to a cis regulatory element, such as a promoter. Preferably, the promotor operatively linked to the first nucleotide sequence is P*_{eutR}*. Preferably, the promotor operatively linked to the second nucleotide sequence is P*_{eutB}*. Both promoters are located in the second nucleotide sequence.

A transcription factor may be any agent that promotes transcription of a gene, such as the target gene. A transcription factor may activate/facilitate transcription by promoting interaction between RNA polymerase and DNA. A transcription factor may be a sequence specific-DNA binding protein that modulates the transcription of DNA into mRNA. A transcription factor may promote transcription by directly or indirectly recruiting RNA polymerase to the promoter of a gene to be transcribed.

The transcription factor may bind to a DNA-binding site, the operator, specific for the transcription factor. The DNA-binding site may be downstream of the nucleotide sequence of the transcription factor, upstream or act in trans. The DNA-binding site may be upstream of the target gene, such as within the promoter of the target gene. The transcription factor may bind to or interact with the DNA-binding site in the absence of the effector. The effector may increase binding of the transcription factor to a DNA-binding site. Expression of the target gene may be leaky. Thus, the target gene may be expressed in the absence of the effector. Expression of the target gene may be increased by binding of the effector.

According to one embodiment of the invention, the transcription factor is the transcription factor, EutR. The nucleotide sequence encoding EutR is referred to herein as SEQ ID NO. 1, as follows:

1 ATGGAAAAAT GGCGGGAAGC GGTTGAGCAC GCCCGTGGCG GCGAGTCCAA GTACAAGATT
61 CTTGTCCAAG CGATCAGCTC GGACATCGAG CAGCATAAGC TGGCGGATGA TCAGCGCCTG
121 CCACCTCAAC GGCAGGTTGC CGATGCCTTG GGGATCAGTG TCCAAACCGT GACAAACGCC
181 TACAAGGAAC TCGAGCGCCA AGGGCTAGTA AGGTGCGAAG TGGGTCGCGG GAGTTTCGTT
241 TCTCGGCGAA TGAGTGACCG TGTAGCAAGC TACATTCTCG ATGCTCCCGA GCGCGCACTC
301 GTCGATCTAT CCACCGCCCG AATCATTCAC ACCCCAGAGC ATGACAGGTA CTGGCGAGAA
361 ACATGCGCCG AGCTTGCTAA TGAGGTCGAT CAAAGCTGGA TCCGACTGTG CCGACCTGTA
421 GCGGGCTTCG ATGCTCATCG TGATGCCGCC TGCCATTGGC TTGGGCGTCT TGGATTGCCA
481 GGGCTGGCTC GAGAAGATGT GTTGATCACA AATGGGGCGG CCCACGGGCT ATTCCTGGCG
541 CTGGCCTCCC TTGCAGGCCC TGATGACCTG GTGCTCTGTG AAGGCATGAC CGATCACGGT
601 GTCATTGGGA ACTCTCAAGT CCTCGGCTTC AATCTGAAAG GCCTAGACAC TGATCGTGAC
661 GGGATTGATC CCGAGCACTT TGAGGACATC TGCAGCAACG AGCGCGTTAC GGCCCTGGTA
721 TGCACGCCGA ACCTTAATAA CCCGACCAGC GCGTTGATGC CCAATAGCCG CAGGAAAGAG
781 ATAGCCTCCA TCGCTCGTCG CTTCGGTGTC CACATTATTG AGGATGACGT CTACGGCCCC
841 CTGCTTAACC AATACGAAGG CCCTCCAATC AGCTCATATG CCCCCGAGCT CTCGTTTTAC
901 TGCACCAGCA TGACGAAGTC AGTTCTGACT GGGCTACGAA TCGGGTTCCT CAAAGTACCT
961 AAACGACTGG CGCTGCGAAC AGAAAGCATC CTTCGCGTGA CGAGCTGGAT GGCAGCCCCC
1021 ATGCTTGCCG AGGTTGCCGC ACGGTGGATT ATGGATGGGA GGGCCGACAC ATTGATCCAA
1081 TTGCAGCGAA GGCTCCTAGA GACACGCCAG AAGCTGGTAG CAGACGAGAT GGGGCGCTAC
1141 CTCTATTGCC ATCGCCCTCA TTCGTTAAAC GCTTGGCTGA ATGTGCCAGC ACATTGGGAT
1201 GTTTCCAGCC TGGTGCGCAC ACTACGACAA CAGCATATTG CGGTTACGTC ACCGGAGCCG
1261 TTCACCGTTC GGGGGGTGAC CCCAGCACGC GGCATCCGGC TTTGCATCGG CGCTGAAACG

1321 AGTGAAAGCG AGCTATCCCA GGCCCTGAAA ACCATGCGGA TGACTTTTGA
ACAATACCCA

1381 GGAGTCCATG ACTTCTGA

Accordingly, the transcription factor is encoded by a nucleotide sequence substantially as set out in SEQ ID NO. 1, or a variant or fragment thereof.

The promoter P*_{eutB}* is an inducible promoter operably linked to a coding region, wherein the coding region encodes a target gene; the transcription factor EutR through the binding to the DNA-binding site, the operator, regulates transcription of the target gene.

The inducible promoter of the inducible gene expression system comprises a nucleotide sequence encoding an operator and promoter domains that can be used to control the transcription of the coding region. The operator may be a DNA-binding site, whereas the promoter may be RNA polymerase-binding site. The operator may be located upstream or downstream to the promoter. The consensus sequence of the operator varies depending on transcription factor.

The operator O*_{eutB}*, DNA-binding site of the transcription factor (e.g. EutR), may be adjacent to the promoter P*_{eutB}* of the target gene. The DNA-binding site of EutR is preferably between positions -50 and -35 (relative to the transcriptional start site of the target gene). According to another embodiment of the invention, the DNA-binding site of EutR is referred to herein as SEQ ID NO. 2, as follows:

### ATTGCCCTAGTACATT

Accordingly, the second nucleotide sequence encodes a DNA-binding site of transcription factor EutR that is substantially as set out in SEQ ID NO. 2, 3, 4, 5, 6, 7, 8, 9, 10, 11, 12, 13, 14 and 15 or a variant or fragment thereof.

A second nucleotide sequence, containing regulatory elements including a DNA-binding site and a promoter, is substantially as set out in SEQ ID NO. 2, 3, 4, 5, 6, 7, 8, 9, 10, 11, 12, 13, 14 and 15 or a variant or fragment thereof.
SEQ ID NO. 3, as follows:
   ATTGCCCTAGTACATTCATTATGACAATCAGCGGCTCGTATAAT
SEQ ID NO. 4, as follows:
   ATTGCCCTAGTACATTCCCTTTTAATCATCCGGCTCGTATAAT
SEQ ID NO. 5, as follows:
   ACGGGTGGCGATTGCCCTAGTACATTCATTATGACAATCAGCGGCTCGTAGACT
SEQ ID NO. 6, as follows:
   ACGCCACCGCTAACGCCTAGTACATTCATTATGACAATCAGCGGCTCGTAGACT
SEQ ID NO. 7, as follows:
   ATTGCGGATCAACATTCATTATGACAATCAGCGGCTCGTAGACT
SEQ ID NO. 8, as follows:
   ATTGCCCTAGTTGTAAGATTATGACAATCAGCGGCTCGTAGACT
SEQ ID NO. 9, as follows:
   ATTGCCCTAGTACATTCTAATACACAATCAGCGGCTCGTAGACT
SEQ ID NO. 10, as follows:
   ATTGCCCTAGTACATTCATTATGTGTTAGAGCGGCTCGTAGACT
SEQ ID NO. 11, as follows:
   ATTGCCCTAGTACATTCATTATGACAATCTCGCCGTCGTAGACT
SEQ ID NO. 12, as follows:
   ATTGCCCTAGTACATTCATTATGACAATCAGCGGCAGCATCACT
SEQ ID NO. 13, as follows: 
ATTGCCCTAGTACATTCATTATGACAATCAGCGGCTCGTAGTGAGACAAAAAAACAATCGTT
CGGTGGTCTCATATG
SEQ ID NO. 14, as follows: 
ATTGCCCTAGTACATTCATTATGACAATCAGCGGCTCGTAGACTCTGTTTTTTACAATCGTT
CGGTGGTCTCATATG
SEQ ID NO. 15, as follows:

ATTGCCCTAGTACATTCATTATGACAATCAGCGGCTCGTAGACTCTGAAAAAATGTTAGGTT
CGGTGGTCTCATATG

The bacterial promoter contains two short sequence elements approximately 10 (-10 element) and 35 (-35 element) nucleotides upstream from the transcription start site. The promoter sequence is recognized by RNA polymerase holoenzyme containing sigma factor. In proteobacteria the consensus sequences of -10 and -35 elements may be [Harley, C.B. and Reynolds, R.P. (1987) Nucleic Acids Res. 15, 2343-2361]:
- 10 element: 5'-TATAAT-3'
- 35 element: 5'-TTGACA-3'

In embodiments in which the inducible gene expression system positively regulates transcription of the coding region, the DNA-binding site may, in the absence of an effector, be transcription factor-free, thus in the passive state. The transcription factor, which acts as an activator, is unavailable to activate binding of RNA polymerase to the RNA polymerase-binding site. Binding of the effector to the transcription factor, enables transcription factor binding to the DNA-binding site activating the RNA polymerase biding to the DNA-binding site and thus transcription of the coding region into a mRNA.

The target gene may be any gene of interest, but it is preferably a reporter gene, which can be used to monitor inducible system response to ectoine in a quantitative manner.

The reporter gene encodes fluorescent protein and is used to monitor gene expression as a fluorescence output. The fluorescent protein is red fluorescent protein or any fluorescent protein suitable for use as a reporter, or a homolog or functional variant thereof.

The biosensor may increase the level of target gene expression by up to 20-fold. In an uninduced state, it exhibits non-detectable background expression.

In this context the term "background expression" refers to the level of protein produced by a target gene in a cell under normal circumstances when expression of the gene is not desired, or expression of the target gene is not activated with an effector. This level of expression is also sometimes referred to as the "leaky" level of expression, occurring because the gene promoter allows some expression even when not specifically activated.

In the biosensor of the invention preferably the dynamic range of expression between the inducible promoter being off and the inducible promoter being on, and gene expression being activated, is high. The dynamic range may be between about 100 and about 1000 fold of the off level, assuming that the off level is detectable. Preferably the dynamic range is between about 100-fold that of the off level.

The inducible gene expression system of the biosensor is composed of a gene encoding transcription factor and inducible promoter, where the transcription factor through the binding to the inducible promoter regulates transcription. The inducible promoter is operably linked to a coding region and is 5' to the coding region.

The inducible gene expression system of the biosensor may modulate transcription of a coding region to which it is operably linked in response to contact of the transcription factor with an effector. The biosensor may modulate transcription of the coding region, in response to contact with an effector, by positively regulating transcription of the coding region (i.e. promoting transcription of the coding region). In a preferred embodiment the effector activates the biosensor such that it promotes transcription of the coding region.

The inducible gene expression system of the biosensor may be activated by a natural or a non-natural agent which acts as the effector.

The inducible gene expression system may be a naturally-occurring biosensor or of a synthetic nature. A naturally occurring inducible gene expression system may be a device responsive to 3-hydroxypropionate, itaconate, indole, lactic acid, arabinose, etc.

The transcription factor of the inducible gene expression system may specifically bind the effector, such as, ectoine, and thus be referred to as an ectoine-responsive inducible gene expression system. Ectoine is 1,4,5,6-tetrahydro-2-methyl-4-pyrimidinecarboxylic acid that has high affinity for the transcription factor EutR of the ectoine-responsive inducible gene expression system.

The ectoine-monitoring biosensor according to the invention is highly beneficial because this device can be used to control gene expression in ectoine dependent manner and to measure exogenous and endogenous ectoine concentration.

The biosensor may comprise a) a gene coding transcription factor suitable to regulate transcription, b) an inducible promoter suitable for use in a prokaryotic host, and c) the coding region for a target gene. The a) and b) form the inducible gene expression system.

The biosensor of the invention may be introduced into a host cell by using any suitable means, such as endocytic uptake, microinjection, ballistic bombardment, a particle gun, electroporation, transduction, transfection, infection or cell fusion. Preferably, the genetic construct is introduced into the cell by using a vector.

Thus, according to another aspect of the invention, there is provided a vector comprising the biosensor of the invention.

The vector may be a recombinant vector. The vector may be a virus, a virus-like particle, a plasmid, a cosmid, a phage, a transposon or a liposome.

According to another aspect of the invention, there is provided a host cell comprising the biosensor according to the invention or a vector according to the invention.

The host cell may be a bacterium, a plant, an algae, a fungi or a protozoa. Preferably, the cell is a bacterium.

The bacterium may be a Gram-negative bacteria. The bacteria may be of the genus Pseudomonas, or Escherichia. The bacterium may be Escherichia coli.

The bacterial cell may be any bacterial species, but preferably members of the bacterial phylum Pseudomonadota, the class Gammaproteobacteria.

The bacterium may be within the order of Aeromonadales, Alteromonadales, Chromatiales, Enterobacterales, Legionellales, Methylococcales, Oceanospirillales, Pasteurellales, Pseudomonadales, Vibrionales, and Xanthomonadales. Preferably, the bacterium is within the order of Pseudomonadales or Enterobacterales.

Within the order Pseudomonadales is the genus, Pseudomonas. Preferred species is P. putida.

Within the order Enterobacterales is genus Escherichia. Preferred species is E. coli.

In a preferred embodiment the host cell is a Pseudomonas or Escherichia cell. Whilst the majority of these species are studied for independent purposes, the emerging field of synthetic biology brings them all together under the same scope - the engineering of novel strains with new functionalities. These designated novel strains are on the one hand facilitating the study of fundamental biological processes and on the other hand, they are advancing biotechnological applications. Such applications include the production of platform chemicals and biofuels (e.g., Pseudomonas putida and Escherichia coli. No studies regarding the application of ectoine-inducible gene expression systems in Pseudomonas and Escherichia have been reported.

According to one aspect of the invention, there is provided (a nucleotide sequence encoding) a DNA-binding site for transcription factor EutR, which has a positive regulatory activity.

A DNA-binding site having regulatory activity may comprise a nucleotide sequence substantially as set of in SEQ ID NO. 2, or a variant or fragment thereof.

According to another aspect, there is provided a biosensor comprising or consisting of a DNA-binding site according to the invention.

Advantageously, the DNA-binding site, the operator, and biosensor according to the invention may be used to tightly regulate the expression of a target gene, particularly a target gene that exhibits leaky expression.

According to another aspect of the invention, there is provided a genetic construct comprising or consisting of a nucleotide sequence encoding a DNA-binding site for transcription factor EutR and a transcription factor EutR,
wherein the DNA-binding site and the transcription factor interacts and promotes transcription of the target gene in response to an effector, e.g. ectoine.

The biosensor according to the invention is advantageous because it may be used to tightly control gene expression of a target gene. Furthermore, the biosensor may be introduced into a another inducible gene expression system comprising a target gene, whose transcription is promoted by the transcription factor, or whose transcription is repressed by the transcriptional repressor in order to add a layer of control over basal transcription of the target gene (i.e. transcription of the target gene in the absence of an effector).

According to yet another aspect of the invention, there is provided at least one genetic construct comprising or consisting of:
a first nucleotide sequence encoding a transcription factor, wherein the transcription factor regulates transcription of the target gene in response to an effector, and
a second nucleotide sequence containing regulatory elements including promoters and a naturally-occurring DNA-binding site for transcription factor. Regulatory elements are a naturally-occurring DNA-binding site and promoter or a synthetic DNA-binding site and promoter; and wherein the regulatory element is a positive regulatory element.
a third nucleotide sequence encoding a target gene, wherein the target gene is expressed in response to an effector and the transcription factor, both of which in complex interacts with the DNA-binding site regulating gene expression.

The naturally-occurring DNA binding site is a Pseudomonas putida KT2440 eutB operator motif OeutB. The synthetic DNA binding site is a modified Pseudomonas putida KT2440 eutB operator motif *OeutB.

The genetic construct according to the invention is advantageous because it provides tight control over a target gene whose transcription is regulated/promoted by a transcription activator, such as a transcription factor. Consequently, the target gene is highly repressed in the absence of any effectors. Thus, little or no expression of the target gene occurs in the absence of an effector.

The term 'in response to' can refer to in response to contact of the cell, biosensor, inducible gene expression system, inducible promoter, vector or transcription factor according to the invention.

The term 'expression' can refer to transcription of a gene.

An inducible gene expression system' can refer to a system that only expresses a target gene in the presence of an effector (i.e. in an ON' state). In the absence of the effector, the system is in an OFF' state.

The term 'regulate(s )' can refer to 'repression' and/or 'activation'. The term 'regulate(s)' can refer to 'inhibition/prevention' and/or 'enhancement/initiation/induction'.

A ` variant or fragment thereof' can be a derivative of (i) the DNA-binding site, the operator, which is capable of biding transcription factor regulating transcription, or, (ii) the transcription factor, or (iii) the target gene, as referred to herein.

### EMBODIMENTS OF THE INVENTION

The following is information describes the materials used, methods and production examples. This information is provided for illustrative purposes and is not intended to limit the scope of the invention.

### Strains, media and growth conditions

All the *Escherichia coli* and *Pseudomonas putida* strains used in this study are listed in Table 1. E. coli TOP 10 was used as a general host for plasmid construction and propagation. All E.coli strains were transformed according a standard protocol and using chemically competent E. coli cells [Sambrook et al (1989) Molecular cloning: a laboratory manual]. All P. putida strains were transformed using electroporation according a standard protocol [Ausubel (2003) Current Protocols in Molecular Biology]. The mixture of electrocompetent cells with 100 ng of plasmid DNA was incubated in electroporation cuvette (0.2 cm gap width, Bio-Rad) on the ice for 5 min, followed by the electroporation at 2.5 kV using a Micropulser (Bio-Rad, Hercules, CA, USA). The transformed cells were recovered by incubation in 1 mL of SOC medium at 30 °C for 2 h and then plated on LB agar with antibiotic followed by incubation at 30 °C for 2 days. E. coli strains were grown at 30 °C, in Luria-Bertani (LB) or Minimal Medium (MM) supplemented with chloramphenicol (25 mg/mL) and P. putida strains were grown at 30 °C, in MM medium supplemented with tetracycline (12.5 mg/mL). MM was composed of 200 mL/L of microelement solution M9 (64 g/L Na₂PO₄·7H₂O, 15 g/L KH₂PO₄, 2.5 g NaCl, 5 g NH₄Cl), 2 mM MgSO₄, 0.1 mM CaCl₂, 1 µg/mL thiamine hydrochloride, 0.4 mM L-leucine and 0.4 % glucose.

### Reagents

All PCR reactions were performed using Phusion Polymerase Master Mix (Thermo Fisher Scientific) or DreamTaq Green PCR Master Mix (Thermo Fisher Scientific). T4 DNA ligase (Thermo Fisher Scientific) was used for DNA ligation reactions. Restriction enzymes were purchased from Thermo Fisher Scientific. Ectoine and hydroxyectoine were purchased from Sigma-Aldrich.

### Plasmid construction

Oligonucleotide primers were synthesized by Thermo Fisher Scientific (Table 2). Plasmids were constructed by restriction enzyme-based cloning procedures. Constructs were verified by DNA sequencing (Eurofins) PCR amplification, or restriction analysis using restriction endonucleases Aatll, BamHi, Bglll, and Ndel (Thermo Fisher Scientific). All plasmids constructed in this study are listed in Table 3. Details of plasmid construction are provided below:

### Fluorescence and Absorbance Measurements

The fluorescence and cell absorbance were measured at multiple time points. Freshly grown bacterial cells were used to inoculate 2 mL of MM containing appropriate antibiotics in 50-mL centrifuge tubes, and cell cultures were grown overnight at 200 rpm and 30 °C. To obtain logarithmically growing cells, *E. coli* and *P. putida* overnight cultures were diluted to 0.05 OD₆₀₀, respectively, in 5 mL of fresh medium with antibiotic. After the cells were grown to an OD₆₀₀ of 0.15-0.2, culture aliquots of 142.5 µL were transferred into flat and clear bottom black 96-well plate (Corning Incorporated) and 7.5 µL of ectoine or hydroxyectoine was added to each culture to the required final concentration. Absorbance and fluorescence of cultures were measured using an Infinite Nano+ microplate reader (Tecan). Multiple time point measurements were taken every 10 min over the course of 15-20 hours with a plate reader measuring RFP fluorescence at excitation and emission wavelengths set to 585 nm and 620 nm, respectively, and absorbance at 600 nm. Obtained fluorescence and absorbance values were corrected by subtracting the autofluorescence and absorbance of the culture medium. The absolute normalized fluorescence was determined dividing the fluorescence value by corresponding absorbance value.

The dose-response of biosensors was determined by using single time-point values of fluorescence and absorbance, which were obtained from the multiple time-point measurements 4 h after supplementation of ectoine or hydroxyectoine at different concentrations ranging from 0 to 5 mM. For all assays, the cultures of E. *coli* and *P. putida* were prepared as described above, and 142.5 µL of logarithmically growing cells, supplemented with 7.5 µL of ectoine or hydroxyectoine, were cultured in 96-well plate. The fluorescence and absorbance were measured using the same conditions and plate reader as indicated above.

### Tables

**Table 1. Bacterial strains used in the study.**

| **Bacterial strain** | **Genotype** | **Reference** |
|---|---|---|
| *Escherichia coli* TOP10 | F⁻ *mcrA* Δ(*mrr-hsdRMS-mcrBC*) Φ80/*acZ*ΔM15 Δ*I*ac*X*74 *recA1 araD*139 Δ(*araleu*)7697 *galU galK rpsL* (StrR) *endA1 nupG* Wild type strain | Thermo Fisher Scientific (Waltham, USA) |
| *Pseudomonas putida* KT2440 | | DSM 6125 (Braunschweig, Germany) |

**Table 2. Oligonucleotide primers used in the study.**

| | |
|---|---|
| **Primer name** | **Primer sequence (5'** → **3')** |
| **P005** | gggcctttcgttttatgacgtcggtcagaagtcatggactcc |
| **P006** | cgtcttcgctactcgccatatgaaatctccctctattgtaattggcc |
| **P028** | gggcctttcgttttatgacgtcaaaaatctccctctattgtaattggcc |
| **P029** | cgtcttcgctactcgccatatgagaccaccgaacgattgtttttttcagagtc |
| **P005Tg** | gggcctttcgttttatgacgtcatcgctatgcttgctcctaagc |
| **P064** | gggcctttcgttttatgacgtccgtttgacacaaatctgaaaaaacgc |
| **EG013** | ccttactcgagtttggatcc |
| **P110** | acaatcagcggctcgtagac |
| **P111** | gtctacgagccgctgattgtcataatgaatgtactagggcaatcgccacccgttttttcagatttgtgtca |
| **P112** | gtctacgagccgctgattgtcataatgaatgtactaggcgttagcggtggcgttttttcag |
| **P113** | gtctacgagccgctgattgtcataatgaatgttgatccgcaatccggtggcg |
| **P114** | gtctacgagccgctgattgtcataatcttacaactagggcaatccggtgg |
| **P115** | gtctacgagccgctgattgtcattgtcaatgtactagggcaatccggt |
| **P116** | gtctacgagccgctgattgtgtattagaatgtactagggcaatccg |
| **P117** | gttcggtggtctcatatggc |
| **P118** | gccatatgagaccaccgaacgattgtttttttcagagtctacgagccgctctaacacataatgaatgta ctagggcaatcc |
| **P119** | gccatatgagaccaccgaacgattgtttttttcagagtctacgacggcgagattgtcataatgaatgta ctagggc |
| **P120** | gccatatgagaccaccgaacgattgtttttttcagagtgatgctgccgctgattgtcataatgaa |
| **P121** | gccatatgagaccaccgaacgattgtttttttgtctcactacgagccgctgattgt |
| **P122** | gccatatgagaccaccgaacgattgtaaaaaacagagtctacgagccgc |
| **P123** | gccatatgagaccaccgaacctaacattttttcagagtctacgagccg |
| **P096** | tcataatgaaccggtggcgttttttcagatttgtg |
| **P097** | acgccaccggttcattatgacaatcagcggctcgtag |
| **P098** | ccgaacgattgtttgaatgtactagggcaatccggtggc |
| **P099** | acattcaaacaatcgttcggtggtctcatatgg |
| **P080** | tcacgaaggtaaaggaggtagttaatggcgagtagcgaagacgttatca |
| **P081** | tacctcctttaccttcgtgatttcagagtctacgagccgctgattgt |
| **P086** | tttccatcatcatatttttactcctaagccacggcctacagcatggacg |
| **P087** | aggagtaaaaatatgatgatggaaaaatggcgggaagcggt |
| **P100** | attgtttttttcagattatacgagccggatgattaaaagggaatgtactagggcaatccgg |
| **P101** | tataatctgaaaaaaacaatcgttcggtg |
| **P102** | attgtttttttcagattatacgagccgctgattgtca |
| **P138** | cattatgacaatcagcggctcgtagact |
| **P139** | agccgctgattgtcataatgaaatgtactagggcaatccggtggc |
| **P140** | agccgctgattgtcataatgaaaatgtactagggcaatccggtggc |
| **P141** | agccgctgattgtcataatgaaaaatgtactagggcaatccggtggc |

**Table 3. Plasmids used in the study.**

| **Plasmid** | **Characteristics** | **Reference** |
|---|---|---|
| **pBRC1** | *Cm*^{r}; mRFP reporter plasmid; P*_{arac}-araC-*T*_{rrnB1}* and P*_{araBAD}-T7*sl*-Ec*RBS*-rfp-*T*_{dbl}* | assembled as described in (Hanko, et al., 2017). |
| **pEct-2** | *Cm*^{r}; P*_{eutR}-eutR-T_{rrnB1}* and P_{ehuB}-*rfp*-T*_{dbl}* from *P. putida* KT2440genomic DNA | This study |
| **pEct-2(T)** | *Tc*^{r}; P*_{eutR}-eutR*-*T_{rrnB1}* and P*_{ehuB}-rfp-*T*_{dbi}* from *P. putida* KT2440 genomic DNA | This study |
| **pEct-2IR** | *Cm*^{r}; P*_{ehuB}*-*rfp*-T*_{dbl}* from *P. putida* KT2440 genomic DNA | This study |
| **pEct-2IR(T)** | *Tc*^{r}; P*_{ehuB}-rfp*-T*_{dbl}* from *P. putida* KT2440 genomic DNA | This study |
| **pEct-1** | *Cm*^{r}; P*_{eutR}-eutR-T_{dbl}* and P*_{eutB}*-*rfp*-T*_{dbl}* from *P. putida* KT2440 genomic DNA | This study |
| **pEct-1(T)** | *Tc*^{r}; P*_{eutR}-eutR-*T*_{dbl}* and P*_{eutB}*-*rfp*-T*_{dbl}* from *P. putida* KT2440 genomic DNA | This study |
| **pEct-1IR** | *Cm*^{r}; P*_{eutB}*-*rfp*-T*_{dbl}* from *P. putida* KT2440 genomic DNA | This study |
| **pEct-11R(T)** | *Tc*^{r}; P*_{eutB}-rfp-*T*_{dbl}* from *P. putida* KT2440 genomic DNA | This study |
| **pEct-1F1** | pEct-1 with P*_{eutB}-rfp-*T*_{dbl}* anternations of replacing 5'-CCACCG with 5'-GGTGGC. | This study |
| **pEct-1F2** | pEct-1 with P*_{eutB}-rfp-*T*_{dbl}* anternations of replacing 5'-GATTGC with 5'-CTAACG. | This study |
| **pEct-1F3** | pEct-1 with P*_{eutB}-rfp-*T*_{dbl}* anternations of replacing 5'-CCTAGT with 5'-GGATCA. | This study |
| **pEct-1F4** | pEct-1 with P*_{eutB}-rfp-*T*_{dbl}* anternations of replacing 5'-ACATTC with 5'-TGTAAG. | This study |
| **pEct-1F5** | pEct-1 with P*_{eutB}-rfp-*T*_{dbl}* anternations of replacing 5'-ATTATG with 5'-TAATAC. | This study |
| **pEct-1F6** | pEct-1 with P*_{eutB}-rfp-*T*_{dbl}* anternations of replacing 5'-ACAATC with 5'-TGTTAG. | This study |
| **pEct-1F7** | pEct-1 with P*_{eutB}-rfp-*T*_{dbl}* anternations of replacing 5'-AGCGGC with 5'-TCGCCG. | This study |
| **pEct-1F8** | pEct-1 with P*_{eutB}-rfp-*T*_{dbl}* anternations of replacing 5'-TCGTAG with 5'-AGCATC. | This study |
| **pEct-1F9** | pEct-1 with P*_{eutB}-rfp-*T*_{dbl}* anternations of replacing 5'-ACTCTG with 5'-TGAGAC. | This study |
| **pEct-1F10** | pEct-1 with P*_{eutB}-rfp-*T*_{dbl}* anternations of replacing 5'-AAAAAA with 5'-TTTTTT. | This study |
| **pEct-1F11** | pEct-1 with P*_{eutB}-rfp-*T*_{dbl}* anternations of replacing 5'-ACAATC with 5'-TGTTAG. | This study |
| **pEct-1F1(T)** | pEct-1(T) with P*_{eutB}-rfp-*T*_{dbl}* anternations of replacing 5'-CCACCG with 5'-GGTGGC. | This study |
| **pEct-1F2(T)** | pEct-1(T) with P*_{eutB}-rfp-*T*_{dbl}* anternations of replacing 5'-GATTGC with 5'-CTAACG. | This study |
| **pEct-1F3(T)** | pEct-1(T) with *P_{eutB}-tip-T_{dbl}* anternations of replacing 5'-CCTAGT with 5'-GGATCA. | This study |
| **pEct-1F4(T)** | pEct-1(T) with P*_{eutB}-rfp-*T*_{dbl}* anternations of replacing 5'-ACATTC with 5'-TGTAAG. | This study |
| **pEct-1F5(T)** | pEct-1(T) with P*_{eutB}-rfp-*T*_{dbl}* anternations of replacing 5'-ATTATG with 5'-TAATAC. | This study |
| **pEct-1F6(T)** | pEct-1(T) with P*_{eutB}-rfp-*T*_{dbl}* anternations of replacing 5'-ACAATC with 5'-TGTTAG. | This study |
| **pEct-1F7(T)** | pEct-1(T) with P*_{eutB}-rfp-*T*_{dbl}* anternations of replacing 5'-AGCGGC with 5'-TCGCCG. | This study |
| **pEct-1F8(T)** | pEct-1(T) with P*_{eutB}-rfp-*T*_{dbl}* anternations of replacing 5'-TCGTAG with 5'-AGCATC. | This study |
| **pEct-1F9(T)** | pEct-1(T) with P*_{eutB}-rfp-*T*_{dbl}* anternations of replacing 5'-ACTCTGwith 5'-TGAGAC. | This study |
| **pEct-1F10(T)** | pEct-1(T) with P*_{eutB}-rfp-*T*_{dbl}* anternations of replacing 5'-AAAAAA with 5'-TTTTTT. | This study |
| **pEct-1F11(T)** | pEct-1(T) with P*_{eutB}-rfp-*T*_{dbl}* anternations of replacing 5'-ACAATC with 5'-TGTTAG. | This study |
| **pEct-1P1** | pEct-1 with P*_{eutB}-rfp-*T*_{dbl}* anternations of replacing 5'-TAGACT with 5'-TATAAT. | This study |
| **pEct-1P2** | pEct-1 with P*_{eutB}-rfp-*T*_{dbl}* anternations of replacing 5'-TTCATTATGACAATCAGCGGCTCGTAGACT with 5'-TTCCCTTTTAATCATCCGGCTCGTATAAT. | This study |
| **pEct-1P1(T)** | pEct-1(T) with P*_{eutB}-rfp-*T*_{dbl}* anternations of replacing 5'-TAGACT with 5'-TATAAT. | This study |
| **pEct-1P2(T)** | pEct-1(T) with P*_{eutB}-rfp-*T*_{dbl}* anternations of replacing 5'-TTCATTATGACAATCAGCGGCTCGTAGACT with 5'-TTCCCTTTTAATCATCCGGCTCGTATAAT. | This study |
| **pEct-1RBS1** | pEct-1 with P*_{eutB}-rfp-*T*_{dbl}* anternations of replacing 5'-CAAGCATAGCG with 5'-TAAAAATAGT. | This study |
| **pEct-1RBS2** | pEct-1 with P*_{eutB}-rfp-*T*_{dbl}* anternations of replacing 5'-AAAACAATCGTTCGGTGGTCTACC with 5'-TCACGAAGGTAAAGGAGGTAGTTA. | This study |
| **pEct-1RBS3** | pEct-1 with P*_{eutB}-rfp-*T*_{dbl}* with anternations from both pEct-1G and pEct-1 R. | This study |
| **pEct-1RBS1(T)** | pEct-1(T) with P*_{eutB}-rfp-*T*_{dbl}* anternations of replacing 5'-CAAGCATAGCG with 5'-TAAAAATAGT. | This study |
| **pEct-1RBS2(T)** | pEct-1(T) with P*_{eutB}-rfp-*T*_{dbl}* anternations of replacing 5'-AAAACAATCGTTCGGTGGTCTACC with 5'-TCACGAAGGTAAAGGAGGTAGTTA. | This study |
| **pEct-1RBS3(T)** | pEct-1(T) with P*_{eutB}-rfp-*T*_{dbl}* with anternations from both pEct-1RBS1 and pEct-1 RBS2. | This study |
| **pEct-1T1** | pEct-1 with P*_{eutB}-rfp-*T*_{dbl}* anternations of T insertion towards 5' from -35 nucleotide, 5'-TTCATT becoming 5'-TTTCATT. | This study |
| **pEct-1T2** | pEct-1 with P*_{eutB}-rfp-*T*_{dbl}* anternations of TT insertion towards 5' from -35 nucleotide, 5'-TTCATT becoming 5'-TTTTCATT. | This study |
| **pEct-1T3** | pEct-1 with P*_{eutB}-rfp-*T*_{dbl}* anternations of TTT insertion towards 5' from -35 nucleotide, 5'-TTCATT becoming 5'-TTTTTCATT. | This study |
| **pEct-1T1(T)** | pEct-1(T) with P*_{eutB}-rfp-*T*_{dbl}* anternations of T insertion towards 5' from -35 nucleotide, 5'-TTCATT becoming 5'-TTTCATT. | This study |
| **pEct-1T2(T)** | pEct-1(T) with P*_{eutB}-rfp-*T*_{dbl}* anternations of TT insertion towards 5' from -35 nucleotide, 5'-TTCATT becoming 5'-TTTTCATT. | This study |
| **pEct-1T3(T)** | pEct-1(T) with P*_{eutB}-rfp-*T*_{dbl}* anternations of TTT insertion towards 5' from -35 nucleotide, 5'-TTCATT becoming 5'-TTTTTCATT. | This study |
| **pEct-1del1** | pEct-1 with P*_{eutB}-rfp-*T*_{dbl}* anternation of removing 5'-ATTGCCCTAGTACA. | This study |
| **pEct-1del2** | pEct-1 with P*_{eutB}-rfp-*T*_{dbl}* anternation of removing 5'-ATTATGACAATCAGCGGCTCGTAGACTCTGAAAA. | This study |
| **pEct-1del1(T)** | pEct-1(T) with *P_{eutB}-tip-T_{dbl}* anternation of removing 5'-ATTGCCCTAGTACA. | This study |
| **pEct-1del2(T)** | pEct-1(T) with *P_{eutB}-tip-T_{dbl}* anternation of removing 5'-ATTATGACAATCAGCGGCTCGTAGACTCTGAAAA. | This study |

### Examples

### Example 1

### EutR transcription factor ectoine biosensor

An ectoine-responsive inducible gene expression system was identified in Pseudomonas putida KT2440 (Figure 1). This system is comprised of gene encoding a transcription factor EutR and intergenic region eutR-eutB. Specifically, the plasmids pEct-1 and pEct-2 were constructed using a sequence and ligation independent cloning protocol (Li & Elledge (2007) Nat. Meth. 4, 251-256). The E. coli eutR gene and intergenic region eutR-eutB was amplified from P. putida strain KT2440 chromosome using oligonucleotide primers P028 (5-GGGCCTTTCGTTTTATGACGTCAAAAATCTCCCTCTATTGTAATTGGCC-3') and P029 (5'-CGTCTTCGCTACTCGCCATATGAGACCACCGAACGATTGTTTTTTTCAGAGTC-3'). In separate reaction the intergenic region eutR-eutB was amplified from P. putida strain KT2440 chromosome using primers P064 (5-GGGCCTTTCGTTTTATGACGTCCGTTTGACACAAATCTGAAAAAACGC-3') and P029 (5'-CGTCTTCGCTACTCGCCATATGAGACCACCGAACGATTGTTTTTTTCAGAGTC-3'). The resulting PCR products were subsequently assembled using the standard cloning protocol with a vector backbone housing a pBBR origin of replication, chloramphenicol (Cm) antibiotic resistance marker, and gene encoding for red fluorescent protein (RFP); forming plasmids pEct-1 (Figure 2) and pEct-2, respectively.

The plasmid pEct-1 was used to characterize biosensor's response to exogenously added ectoine. Experiments were performed to determine the kinetics of induction establishing the time that is required for the system to respond to a change in the ectoine and the dose response of the system. To evaluate biosensor's response to the ectoine, the fluorescence output was quantified.

First, the time course experiments were performed in E. coli and P. putida. Accordingly, cells of both microorganisms harbouring the plasmid pEct-1, were cultivated in minimal medium (MM) at 200 rpm and 30° C (E. coli: Cm 50 mg/L; P. putida: Tet 12.5 mg/L), in the presence and absence of 1 mM ectoine; their optical density (OD600) and RFP fluorescence were monitored over time (Figure 3). In both microorganisms, the RFP expression above the level of the uninduced culture started with 60-90 minutes delay after addition of ectoine (Figure 3 and Figure 4) supporting the notion that the ectoine's catabolic product, the alpha-ADABA, as identified previously [Schultz et al (2017) Environ. Microbiol. 19(11), 4599-4619; Hermann et al (2021) Front. Microbiol. 12, 764731] is the effector that activates gene expression in P. putida. The fluorescence continued to increase throughout the course of 20-hour time course experiment. Importantly, the uninduced baseline (no ectoine) remained at 0 fluorescence units. A biosensor with low baseline activity would be desirable as it offers gene expression control system which reduces or even eliminates background levels of gene expression and ensures a tight regulation of gene expression. Such "non-leaky" system helps improving the quantification accuracy of reporter fluorescence, but also can be used to control the gene expression that can be harmful or even toxic to a cell.

Next, the dose-response profile of the biosensor using different concentrations of ectoine was conducted. E. coli and P. putida harbouring plasmid pEct-1 was cultured overnight in MM (Cm 50 mg/L and Tet 12.5 mg/L, 200 rpm, 30° C). Cultures were then inoculated 5% v/v into fresh MM media (Cm 50 mg/L), grown until final cell densities reached an absorbance of 0.20 at 600 nm and subsequently mixed 19:1 with fresh media supplemented with ectoine to the final volume of 150 mL in 96 deep-well plates. The ectoine final concentration ranged from 0 to 5 mM. The cultures were grown for 10 hours (200 rpm, 30° C.), at which point the absorbance at 600 nm and RFP fluorescence were measured. The RFP fluorescence values (relative fluorescent units) are normalized relative to the absorbance measurements and plotted versus the ectoine concentration. A dose-response curves for E. coli- and P. putida-based biosensor are provided in Figure 5 and Figure 6, respectively. The biosensor showed linear fluorescence response to the ectoine when the ectoine is exogenously present in the range of 1-20 µM, demonstrating limit of detection below 1 µM, which is comparable to the most superior method reported using reversed phase-HPLC [Chen et al (2019) Nat. Prod. Res. 33(8), 1122-1126].

### Example 2

### Identification of regulatory elements of ectoine biosensor

In bacterial gene expression, an RNA polymerase (RNAP) and transcription initiation factors bind to the promoter sequence including -10 (consensus sequence TTGACA) and -35 (TATAAT) sequence elements in a sequence-specific fashion forming an RNAP-promoter complex that initiates transcription at the transcription start site (TSS), position +1. Using intergenic region sequence eutR-eutB, the putative -10 and -35 sequence elements of promoter P*_{eutB}* and TSS were manually determined. It should be noted however, the determined -10 and -35 elements is only prediction that would require an experimental validation by the analysis of every nucleotide residue present in these regulatory elements.

Regulation of gene expression in response to the effector (i.e. ectoine) is commonly mediated by transcription factor that usually interacts directly with the effector or its catabolic product (i.e. alpha-ADABA). Transcription factor interacts with conserved cis-acting element, the operator sequence, which is often located in close proximity to or within the promoter's -10 and -35 sequence elements. Frequently, the promoter drives the expression of genes involved in the metabolism of the effector. Since eutR encodes the transcription factor that regulates the gene expression in the ectoine-responsive system, the operator sequence(s) required for the interaction with transcription factor/regulation must be situated in close proximity to the promoter P*_{eutB}*. A simple approach to identify regulatory elements within the promoters of regulated genes is to compare sequences from divergent species. By multiple alignment, conserved elements can be distinguished from sequences that have evolved more rapidly. Accordingly, an approach to determine transcription factor-binding motif was chosen. The rationale of this approach was based on the notion that operator sequences will be conserved among those species that have conserved genomic context relevant to the transcription factor (i.e. EutR) that controls ectoine catabolic regulon. In this sense, only transcription factors with more than 70% identity and adjacent intergenic regions were considered. Considering that EutR (accession number WP_029886623), can be classified as a member of MocR/GabR sub-family of GntR-type transcription factors, a total of 32 non-redundant GntR-type transcription factors from taxonomically related Pseudomonadales were aligned and grouped through a Neighbor Joining algorithm using the program MEGA version 6 (Figure 7).

The intergenic region eutR-eutB of the most phylogenetically related strains based on EutR protein sequence were used to identify consensus motifs using the WebLogo application [Crooks et al (2004) Genome Res. 14, 1188-1190]. As a result, a consensus motif of 18 bp in length and located between positions -50 and -33 in respect to the TSS, termed O*_{eutB}* was identified as hypothetical regulatory element of the ectoine biosensor (Figure 8). Figure 9 illustrates the EutR/OP*_{eutB}* system from P. putida strain KT2440 and the arrangement and positioning of genes eutR and eutB, as well as of putative operator O*_{eutB}* and promoter P*_{eutB}*.

Following the identification of putative operator O*_{eutB}* and promoter P*_{eutB}*, two new variants were constructed in which either O*_{eutB}* or P*_{eutB}* were deleted, generating plasmid constructs pEct-1 del1 and pEct-1del2, respectively. The data obtained using pEct-1del1 and pEct-1del2 (Figure 10) confirmed that O*_{eutB}* and P*_{eutB}*, are a key regulatory elements for the functionality of the EutR/OP*_{eutB}* system. The same expression profile was observed with pEct-2 when eutR is absent, indicating that the system requires eutR for the activation of gene expression.

Above data suggested that the putative operator O*_{eutB}* sequence is involved in P*_{eutB}* activation and that the putative -10 and -35 elements are sequence determinants of promoter P*_{eutB}*. Further investigation of the DNA sequence including these regulatory elements was carried out by applying the mutational analysis.

Twelve variants (F1-F12) with mutated six-nucleotide sequences located between positions -57 and +9 in respect to the TSS were generated employing an overlapping PCR strategy [Ho et al (1989) Gene 77(1), 51-59]. The plasmids with mutated variants were assembled by ligation-based cloning [Sambrook et al (1989) Molecular cloning: a laboratory manual] and were named pEct-1F1 to pEct-1F12, whereby sequence blocks of 6-nucleotide were substituted by complementary sequences (Figure 11). The EutR/OP*_{eutB}* system's activity was evaluated by measuring reporter fluorescence of E. coli strains carrying plasmids pEct-1 F1 to pEct-1F12 (Figure 12).

Substitutions of 6-nucleotide sequence F5, F6 and F10 (plasmids pEct-1F5, pEct-1F6 and pEct-1F10, respectively) with positions -33 to -28, -27 to -22 and -3 to +3 had no significant effect on P*_{eutB}* activity, compared to the plasmid pEct1-1 containing unchanged sequence of intergenic region eutR-eutB. The substitutions in positions -57 to -52 and +4 to+9 moderately decreased gene expression (pEct-1F1 and pEct-1 F11), whereas those that were introduced in positions -51 to -46, -45 to -40 and -39 to -34 (pEct-1F2, pEct-1 F3 and pEct-1F4, respectively), as well as those in positions -15 to -10 and -9 to -4 (pEct-1F8 and pEct-1 F9, respectively) had a severe effect on fluorescence output, completely abolishing reporter's expression. Interestingly, substitution in the position -21 to -16 resulted in a significant increase in the reporter gene expression (pEct-1F7). These results support the validity of the previously determined positions of the putative elements -10 and -35, which are mutated in F4, F8 and F9 variants. Importantly, the data obtained using variants F2, F3 and F4 covering positions -50 to -33 agrees with O*_{eutB}* being a regulatory element of the ectoine biosensor as this has been predicted (Figure 13).

The data presented here provides a general outline of the mechanisms behind the EutR/OP*_{eutB}* system from P. putida enabling the generation of a synthetic system with both an improved expression and an enhanced dynamic range.

### Example 3

### Improvement of ectoine biosensor

Having confirmed the importance of operator O*_{eutB}* and promoter P*_{eutB}*, sequences, several new variants were constructed aiming to improve biosensor by increasing expression and enhancing dynamic range of EutR/OP*_{eutB}* system in E. coli-based biosensor. In this respect, first, the EutR/OP*_{eutB}* system was aimed to be improved through either the enhancing ribosome binding site that controls the translation initiation rate of EutB biosynthesis by increasing base-pairing between Shine-Dalgarno (SD) sequence and the anti-SD sequence GAUCACCUCCUUA at the 3' end of 16S ribosomal RNA, the optimising the distance between SD sequence and eutR start codon AUG, or both. For this purpose, variants RBS1 to RBS3 were generated and assembled into the plasmids pEct-1 RBS1 to pEct-1 RBS3. Second, the promoter P*_{eutB}* sequence was mutated by substituting the -10 element (sequence TAGACT) with E. coli consensus sequence TATAAT [Harley and Reynolds (1987) Nucleic Acids Res. 15, 2343-2361] (plasmid pEct-1P1) or by substituting whole promoter core region including -35 to -10 elements and a spacer between these two elements (positions -31 to -3 in respect to the TSS) with a synthetic P13 core sequence [Alagesan et al (2018) Appl. Environ. Microbiol. 84, 19].

The ribosome binding site variants RBS1, RBS2, and RBS3 (Figure 14A), and promoter P*_{eutB}* variants P1 and P2 (Figure 15A) were generated and assembled as plasmids constructs employing an overlapping PCR strategy [Ho et al (1989) Gene 77(1), 51-59] and ligation-based cloning [Sambrook et al (1989) Molecular cloning: a laboratory manual]. E. coli strains carrying plasmids pEct-1-RBS1 to pEct-1-RBS3 and pEct-1-P1 to pEct-1-P2 were used to evaluate system's response to the ectoine by measuring reporter's fluorescence.

The optimisation of the distance between SD sequence and eutR start codon AUG (pEct-1 RBS1) showed a more than 2-fold increase of fluorescence output when the ectoine was present exogenously with no baseline activity observed in the absence of ectoine (Figure 14B) if compared to results obtained using plasmid pEct1-1 containing unchanged sequence of intergenic region eutR-eutB. Furthermore, the alteration of promoter P*_{eutB}* sequence by substituting -10 sequence TAGACT with TATAAT (pEct-1-P1) resulted in a more than 10-fold increase of the reporter gene expression compared to results obtained with unchanged sequence of intergenic region eutR-eutB (Figure 15B). In this instance, only a minor increase in the baseline activity was observed in the absence of ectoine. This result supports the validity of the previously determined positions of the putative 10 element. Significantly, the data obtained using variants RBS1 and P1 demonstrate an improved biosensor that exhibits increased reporter expression and enhanced dynamic range of EutR/OP*_{eutB}* system in response to ectoine.

## Claims

1. A method for detecting ectoine, the method comprises
providing a recombinant host cell as biosensor,
**characterized in that**
a first nucleotide sequence of the recombinant host cell is encoding a transcription factor, wherein the transcription factor regulates transcription of the target gene in response to an effector;
a second nucleotide sequence of the recombinant host cell is containing regulatory elements including a DNA-binding site, the operator, transcription of which transcription factor regulates and a promoter;
and a third nucleotide sequence of the recombinant host cell comprises a reporter gene operably linked to the promoter that is activated in response to the effector;
the host cell is cultured under conditions when the effector is exogenously or endogenously present;
the effector interacts with transcription factor by activating transcription of the reporter gene;
the reporter gene is monitored; and
the concentration of ectoine is monitored, where the monitoring of ectoine comprises:
i) providing a host cell which contains a genetic construct, the construct comprising polynucleotide to be transcribed, wherein the polynucleotide comprises a coding region encoding a target gene operably linked to a transcription factor and regulatory elements;
ii) exposing the transformed cell to an effector of the transcription factor thereby effecting expression of the target gene wherein the target gene is reporter gene.

2. The method of claim 1, wherein an effector is an ectoine or hydroxyectoine.

3. The method of claim 1, wherein the transcription factor is a *Pseudomonas putida* KT2440 EutR transcription factor.

4. The method according to any of previous claims, wherein the first nucleotide sequence encoding the transcription factor is substantially as set out in SEQ ID NO. 1, or a variant or fragment thereof; and
wherein a second nucleotide sequence, containing regulatory elements including a DNA-binding site and a promoter, is substantially as set out in SEQ ID NO. 2, 3, 4, 5, 6, 7, 8, 9, 10, 11, 12, 13, 14 and 15 or a variant or fragment thereof; and
wherein a second nucleotide sequence encoding a DNA-binding site of transcription factor EutR, is substantially as set out in SEQ ID NO. 2, 3, 4, 5, 6, 7, 8, 9, 10, 11, 12, 13, 14 and 15 or a variant or fragment thereof.

5. The method according to any of previous claims, wherein regulatory elements are a naturally-occurring DNA-binding site and promoter or a synthetic DNA-binding site and promoter; and wherein the regulatory element is a positive regulatory element.

6. The method according to claim 5, wherein the naturally-occurring DNA binding site is a Pseudomonas putida KT2440 eutB operator motif OeutB.

7. The method according to claim 5, wherein the synthetic DNA binding site is a modified Pseudomonas putida KT2440 eutB operator motif *OeutB.

8. The method according to claims 1, 4, 5, wherein the promoter is a Pseudomonas putida KT2440 PeutB promoter.

9. The method of claim 1, wherein the reporter gene encodes fluorescent protein and is used to monitor gene expression as a fluorescence output.

10. The method according to claim 9, wherein the fluorescent protein is red fluorescent protein or any fluorescent protein suitable for use as a reporter, or a homolog or functional variant thereof.

11. The method according to claim 1, wherein the genetic construct is introduced into the cell by using a vector.

12. The method according to claim 11, wherein a vector is a virus, a virus-like particle, a plasmid, a cosmid, a phage, a transposon or a liposome.

13. The recombinant host cell according to any of previous claims.

14. The recombinant host cell of claim 13, wherein the cell is bacterium.

15. The recombinant host cell according to claim 13, 14, wherein the recombinant host cell is a Pseudomonas putida cell or an Escherichia coli cell.
